# EUROPEAN PATENT APPLICATION

(11) **EP 0 581 108 A2**
(43) Date of publication of application: **02.02.1994**
(21) Application number: 93111232.0
(22) Date of filing: 13.07.1993
(51) Int. Cl.: C12P 21/08, A61K 39/395

(54) **Bispecific antibody reactive with sialyl-Lea antigen and CD3**

(30) Priority: 14.07.1992 JP 186874/92
(71) Applicant: KYOWA HAKKO KOGYO CO., LTD., Chiyoda-ku, Tokyo 100 (JP)
(72) Inventor: Ohta, So, Tokyo (JP); Hanai, Nobuo, Sagamihara-shi, Kanagawa (JP); Nishimura, Takashi, Atsugi-shi, Kanagawa (JP); Habu, Sonoko, Tama-shi, Tokyo (JP)
(74) Representative: Kinzebach, Werner, Dr.

(57) **Abstract**

The present invention relates to a bispecific antibody reactive with sialyl-Le^{a} antigen and CD3, which comprises an anti-sialyl-Le^{a} monoclonal antibody and an anti-CD3 monoclonal antibody. The bispecific antibody is useful for the treatment of cancer.

## Description

### FIELD OF THE INVENTION

This invention relates to bispecific antibodies suitable for use in treating cancer.

### BACKGROUND OF THE INVENTION

One of the serious problems involved in passive tumor immunotherapy is the difficulty in carrying effector cells to a target tumor site. With the development of bispecific antibodies capable of specifically reacting with both killer cells and target cells, attempts have been made to carry antitumor effector cells to target cells (*Nature*, 316, 354 (1985); *Nature*, 314, 628 (1985)). The term "bispecific antibody" as used herein means an antibody capable of specifically recognizing two different antigens, which antibody can be produced chemically or using cell fusion methods.

For example, the bispecific antibodies of the invention can be produced by a process in which two immunoglobulin molecules are linked together using a cross linking agent such as N-succinimidyl 3-(2-pyridyldithiol)-propionate, S-acetylmercaptosuccinic acid anhydride or the like (*J*. *Exp*. *Med*., 163, 166 (1986)) or by a process in which Fab fragments of immunoglobulin molecules are linked together (*Eur*. *J*. *Immunol*., 19, 1437 (1989)).

On the basis of clinical cases and experimental facts, it has been confirmed that a passive tumor immunotherapy, using a bispecific antibody comprising an anti-CD3 monoclonal antibody and an anti-tumor monoclonal antibody, is effective as a means for treating a tumor (*Lancet*, 335, 368 (1990); *Immunol. Today*, 12, 51 (1991)). A bispecific antibody specific for target molecules broadly distributed in tumor cells can be used in the treatment of cancer when combined with passive tumor immunotherapy.

Sialyl-Le^{a} antigen is a carbohydrate antigen which is produced in various human cancer cells, expressed on the membranes of such cells and is secreted extracellularly (*Cancer Research*, 43, 5489 (1983)). Several monoclonal antibodies capable of recognizing the Sialyl-Le^{a} antigen have been reported. For example, an antibody NS19-9 has been reported in *J*. *Biol*. *Chem*., 257, 14365 (1982), CA50 in *British Medical Journal*, 288, 1479 (1984), MSW113 in *J*. *Biochem*., 104, 817 (1988), CC3C195 in *Arch*. *Biochem*. *Biophys*., 225, 214 (1987), Span-1 in *Cancer*, 60, 1636 (1987), and KM231 (AMC-462) in JP-A 63-21562 and in *Anticancer Research*, 8, 329 (1988) (The term "JP-A" as used herein means an "unexamined published Japanese patent application"). Among these monoclonal antibodies, KM231 (AMC-462) has a strong affinity for the sialyl-Le^{a} antigen (*Anticancer Research*, 10, 1579 (1990) and is useful for the treatment of cancer (*Anticancer Research*, 11, 2003 (1991)).

### SUMMARY OF THE INVENTION

An object of the present invention is to provide bispecific antibodies suitable for use in treating cancer.

The present invention provides a bispecific antibody reactive with sialyl-Le^{a} antigen and CD3, which comprises an anti-sialyl-Le^{a} monoclonal antibody, or binding fragment thereof, and an anti-CD3 monoclonal antibody, or binding fragment thereof.

Since sialyl-Le^{a} antigen is produced in large quantities in various human cancer cells, a bispecific antibody specific for both sialyl-Le^{a} antigen and CD3 is expected to be highly effective for clinical treatment of cancer when used in combination with a passive tumor immunotherapy.

### BRIEF DESCRIPTION OF THE DRAWINGS

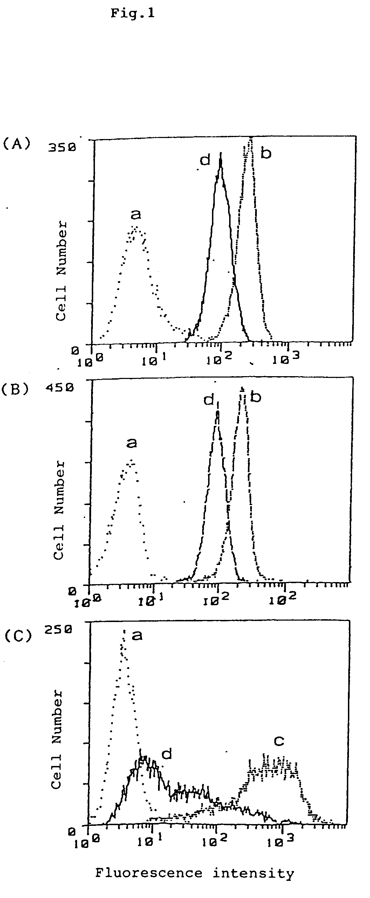

Fig. 1 shows the results of reactivities of antibodies with (A) activated CD4⁺ T cells, (B) activated CD8⁺ T cells and (C) sialyl-Le^{a} antigen-positive cells LS174T examined by fluorescent immunoassay and analyzed by flow cytometry (FACscan), wherein a is a fluorescence pattern obtained without using antibodies, b is a pattern obtained using a monoclonal antibody OKT3, c is a pattern obtained using a monoclonal antibody KM231 and d is a pattern obtained using the bispecific antibody.

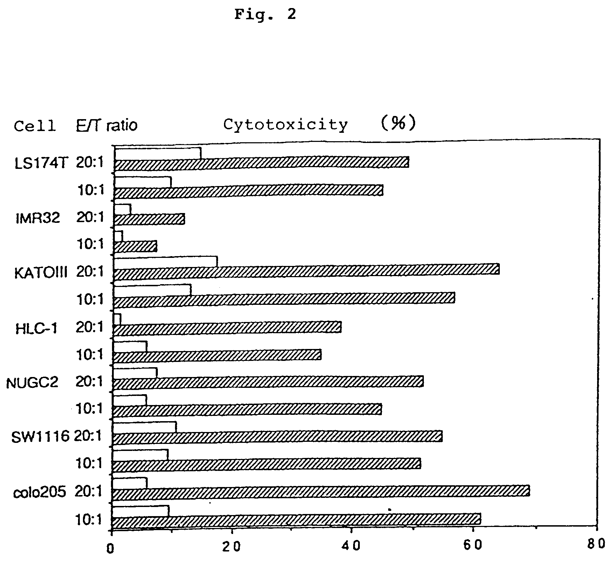

Fig. 2 shows antitumor effects of activated CD8⁺ T cells on sialyl-Le^{a} antigen-positive or negative cells labeled with ⁵¹Cr in the presence or absence of the bispecific antibody, wherein open and oblique columns respectively indicate cytotoxicity in the absence and presence of the bispecific antibody.

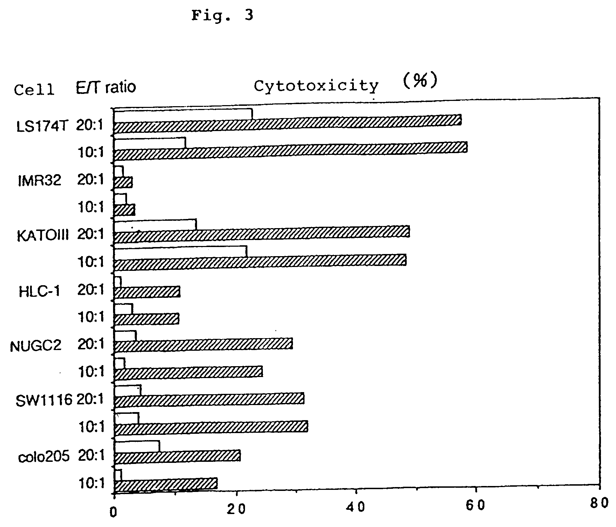

Fig. 3 shows antitumor effects of activated CD4⁺ T cells on sialyl-Le^{a} antigen-positive or negative cells labeled with ⁵¹Cr in the presence or absence of the bispecific antibody, wherein open and oblique columns respectively indicate cytotoxicity in the absence and presence of the bispecific antibody.

### DETAILED DESCRIPTION OF THE INVENTION

The bispecific antibody of the present invention is an antibody having a molecular weight of about 100,000 which is specific for both CD3-positive lymphocytes and sialyl-Le^{a} antigen-expressing cells.

A bispecific antibody of the present invention can be produced in the following manner. That is, a monoclonal antibody specific for a tumor antigen, preferably, the sialyl-Le^{a} antigen, and a monoclonal antibody specific for CD3 is treated with a proteolytic enzyme such as pepsin, papain to obtain the F(ab')₂ fragments of such antibodies each of which fragments is subsequently converted into Fab'-SH, having a free SH-group by reduction of the fragment using dithiothreitol (DTT) or the like. Then, either of the two Fab'-SH fragments is converted into Fab'-S-NB by treating it with 5,5-dithiobis 2-nitrobenzoic acid (DTNB) or the like. The thus prepared Fab'-SH and Fab'-S-NB are mixed together at a ratio of 1:1, and the resulting product is purified by fast protein liquid chromatography (FPLC) and the like, therebobtaining the bispecific antibody.

The anti-sialyl-Le^{a} monoclonal antibody to be used in the present invention may be any monoclonal antibody as long as it is specific for the sialyl-Le^{a} antigen.

A monoclonal antibody specific for the sialyl-Le^{a} antigen may be produced in the following manner. Firstly, animals are immunized with human gastric cancer cells or the like which are expressing the sialyl-Le^{a} antigen at a high level, and splenocytes isolated from the thus immunized animals are fused with mouse myeloma cells to produce hybridoma strains from which a hybridoma is selected that is capable of producing a monoclonal antibody that reacts with sialyl-Le^{a} antigen-expressing cells but not with non-expressing cells. The thus selected hybridoma is cultured in a medium or administered to mice to cause ascites tumor, and the anti-sialyl-Le^{a} monoclonal antibody of interest is purified from the resulting culture or ascitic fluid using a protein A column and the like. Alternatively, the monoclonal antibody may be produced using an established hybridoma strain such as KM231 (JP-A 63-21562 corresponding to EP-A- 0 253 646), NS19-9 (J. Immunol. Methods, 80, 107-116 (1985)) or the like.

The anti-CD3 monoclonal antibody to be used in the present invention may be any monoclonal antibody as long as it is specific for CD3. The CD3 monoclonal antibody specific for CD3 may be produced in the same manner as described above except for using human peripheral blood lymphocytes in place of the cancer cells expressing the sialyl-Le^{a} antigen. Usable as an anti-CD3 monoclonal antibody are OKT3 which can be produced using established hybridoma strain OKT3 (JP-B 63-62520 corresponding to EP-A- 018 795; the term "JP-B" as used herein means an "examined Japanese patent publication", RIV (Transplant. Proc., 21, 1026-1027 (1989)) or the like, with OKT 3 being preferred.

The following is a detailed description of a method of producing the bispecific antibody of the present invention.

### (1) Immunization of animals and preparation of antibody-producing cells:

### (1-1) Preparation of monoclonal antibody specific for sialyl-Le^{a} antigen

Mice or rats of 3 to 20 weeks of age are immunized with the antigen (membrane components of cells expressing the sialyl-Le^{a} antigen at a high level), and antibody-producing cells are collected from the spleen, lymph node or peripheral blood of the animals. The immunization can be carried out by administering the antigen to the animals, subcutaneously, intravenously or intraperitoneally, together with an appropriate adjuvant, such as complete Freund's adjuvant, or aluminum hydroxide gel plus pertussis vaccine.

Following the first antigen administration, the antigen is administered repeatedly 5 to 10 times at one- to two-week intervals. Three to seven days after each administration, a blood sample is taken from each animal from the venous plexus of the fundus of the eye, and the serum derived from the sample blood is tested, for example, by enzyme immunoassay (ELISA method; E. Harlow and D. Lane, *Antibodies* - *A Laboratory Manual*, Cold Spring Harbor Laboratory, 1988] to determine whether it is reactive with the antigen. A mouse or rat whose serum shows a sufficient antibody titer against the antigen used for immunization is submitted for use as a splenocyte source.

### (1-2) Preparation of monoclonal antibody specific for CD3

Mice or rats of 3 to 20 weeks of age are immunized with the antigen (human peripheral blood lymphocytes), and antibody-producing cells are collected from the spleen, lymph node or peripheral blood of the animals. The immunization may be carried out by administering the antigen to the animals, subcutaneously, intravenously or intraperitoneally, together with an appropriate adjuvant, such as complete Freund's adjuvant, or aluminum hydroxide gel plus pertussis vaccine.

Following the first antigen administration, the antigen is administered repeatedly 5 to 10 times at one- to two-week intervals. Three to seven days after each administration, a blood sample is taken from each animal from the venous plexus of the fundus of the eye, and the serum derived from the sample blood is tested by enzyme immunoassay or a similar method to determine whether it is reactive with the antigen. A mouse or rat whose serum shows a sufficient antibody titer against the antigen used for immunization is used as a splenocyte source.

In preparation for fusion between splenocytes and myeloma cells, the spleen of the immunized mouse or rat is excised 3 to 7 days after the final administration of the antigen and splenocytes of the spleen are collected. That is, the spleen is cut to pieces in an MEM medium (Nissui Pharmaceutical Co., Ltd.), and cells are dissociated using a forceps. After centrifugation (1,200 rpm, 5 minutes), the supernatant is discarded, and the sediment is treated with Tris-ammonium chloride buffer (pH 7.65) for 1 to 2 minutes to eliminate erythrocytes. The remaining cells are washed three times with the MEM medium and submitted as splenocytes for cell fusion.

### (2) Preparation of myeloma cells

Mouse-derived established cell lines are used as the myeloma cells. Thus, for instance, the 8-azaguanine-resistant mouse (BALB/c-derived) myeloma cell lines P3-X63Ag8-U1 (P3-U1) (*Eur*. *J*. *Immunol*., 6, 511 (1976)), SP2/O-Ag14 (SP-2) (*Nature*, 276, 269 (1978)), P3-X63-Ag8653 (653) (*J*. *Immunol*., 123, 1548 (1979)) and P3-X63-Ag8 (X63) (*Nature*, 256, 495 (1975)) may be used. These cell lines are subcultured in an 8-azaguanine medium [prepared by supplementing RPMI-1640 medium with glutamine (1.5 mM), 2-mercaptoethanol (5 x 10⁻⁵ M), gentamicin (10 µg/ml) and fetal calf serum (FCS) (CSL; 10%) and further supplementing the resulting medium (to be referred to as "normal medium" hereinafter) with 8-azaguanine (15 µg/ml)]. Three to four days before cell fusion, subculture is performed in the normal medium to ensure a cell number of not less than 2 x 10⁷ cells at the time of cell fusion.

### (3) Cell fusion

The antibody-producing cells obtained by immunization as described in the above step (1) and the myeloma cells obtained as described in the above step (2) are washed well with the MEM medium or PBS (1.83 g of disodium phosphate, 0.21 g of monopotassium phosphate and 7.65 g of sodium chloride per liter of distilled water, pH 7.2), and mixed in a proportion of 5 to 10 antibody-producing cells per myeloma cell, and the mixture is subjected to centrifugation (1,200 rpm, 5 minutes). The supernatant is discarded, the sediment (cells) is thoroughly dissociated, a mixture consisting of 2 g of polyethylene glycol-1,000 (PEG-1,000), 2 ml of MEM and 0.7 ml of dimethyl sulfoxide is added to the cells in an amount of 0.2 to 1 ml/10⁸ antibody-producing cells at 37°C with stirring, then several 1 to 2 ml of MEM are added at 1- to 2-minute intervals, and the whole amount is made 50 ml by further addition of MEM. After centrifugation (900 rpm, 5 minutes), the supernatant is discarded, the cells are loosened gently and then suspended in 100 ml of an HAT medium [prepared by supplementing the normal medium with hypoxanthine (10⁻⁴ M), thymidine (1.5 x 10⁻⁵ M) and aminopterine (4 x 10⁻⁷ M)] by repeated drawing up into and discharging from a graduated pipette.

This suspension is distributed in 100 µl portions into each well of 96-well incubation plates, and incubation is carried out in a 5% CO₂ incubator at 37°C for 7 to 14 days.

After incubation, a portion of the culture supernatant is taken from each well and subjected to enzyme immunoassay or the like, and hybridoma strains thereby selected that are capable of reacting with the sialyl-Le^{a} antigen-expressing cells but not with cells which do not express the sialyl-Le^{a} antigen. Each of the thus selected hybridoma strains is subjected to cloning twice by the limiting dilution method using HT medium (HAT medium minus aminopterine) for the first cloning and normal medium for the second. Strains for which a high antibody titer is constantly observed are selected as anti-sialyl-Le^{a} monoclonal antibody-producing hybridoma strains.

Anti-CD3 monoclonal antibody-producing hybridoma strains can be produced in the same manner.

### (4) Preparation of monoclonal antibodies

The anti-sialyl-Le^{a} monoclonal antibody-producing hybridoma cells and anti-CD3 monoclonal antibody-producing cells obtained in the above step (3) are intraperitoneally injected into 8- to 10-week-old mice or nude mice treated with pristane [by intraperitoneal administration of 0.5 ml of 2,6,10,14-tetramethylpentadecane (pristane) followed by 2 weeks of feeding] at a dose of 2 x 10⁶ to 5 x 10⁷ cells per animal. The hybridoma causes ascitic tumor in 10 to 21 days. The ascitic fluid is collected from the mice, centrifuged (3,000 rpm, 5 minutes) to remove solid matter and, after salting out with 40 to 50% ammonium sulfate, passed through a DEAE-Sepharose column, a protein A column or a Cellulofine GSL 2000 (Seikagaku Kogyo Corporation) column. Thereafter, IgG or IgM fractions are collected to give purified monoclonal antibody.

The subclass of the antibody can be determined using a mouse monoclonal antibody typing kit (Zymed Laboratories) or a rat monoclonal antibody typing kit (Nordic Immunology). The amount of protein can be determined by the Lowry method (J. Biol. Chem., 193, 265 (1951)),
followed by calculation based on the optical density at 280 nm.

### (5) Preparation of bispecific antibody

An F(ab')₂ fragment of an anti-CD3 monoclonal antibody prepared using a proteolytic enzyme such as papain, pepsin or the like (*J*. *Immunol*., 131, 2895 (1983)) is reduced using a reducing agent such as DTT. The reducing reaction is terminated with DTNB, and the thus formed Fab'-S-NB is isolated by a gel chromatography. An F(ab')₂ fragment of an anti-sialyl-Le^{a} monoclonal antibody prepared using papain, pepsin or the like is reduced using a reducing agent such as DTT, and the thus formed Fab'-SH is isolated by a gel chromatography.

Thereafter, the thus prepared Fab'-S-NB and Fab'-SH are mixed together at a ratio of 1:1 and incubated for several hours, and the thus reconstructed F(ab')₂ is purified by gel chromatography to give the bispecific antibody which was submitted to the following experiment.

### (6) Confirmation of the reactivity of bispecific antibody

Reactivities of the bispecific antibody prepared in the above step (5) with human lymphocytes and sialyl-Le^{a} antigen-positive cells are examined for example by a fluorescent antibody technique (E. Harlow and D. Lane, *Antibodies* - *A Laboratory Manual*, Cold Spring Harbor Laboratory, 1988).

### (7) Antitumor effect of bispecific antibody

CD8-positive and CD4-positive T cells are isolated from human peripheral blood lymphocytes using a cell sorter and cultured in the presence of anti-CD3 antibody and interleukin-2 (IL-2), thereby activating lymphocytes (to be referred respectively to as "activated CD8⁺ T cells" and "activated CD4⁺ T cells" hereinafter). Thereafter, the activated CD8⁺ T cells or activated CD4⁺ T cells and sialyl-Le^{a} antigen-positive cells labeled with ⁵¹Cr are cultured in the presence or absence of the bispecific antibody to examine the antitumor effect by measuring ⁵¹Cr gamma rays released from dead cells.

The bispecific antibodies according to the present invention can be used alone as an antitumor agent. They may be formulated into an antitumor composition together with at least one pharmaceutically acceptable carrier. For instance, the bispecific antibodies are dissolved in physiological saline, an aqueous solution of glucose, lactose or mannitol and the like. The powder of the bispecific antibodies for injection can be prepared by lyophilizing the bispecific antibodies in accordance with the conventional method and mixing the lyophilized products with sodium chloride. The antitumor composition may further contain additives conventionally used well known in the art of medical preparation, for example, pharmaceutically acceptable salts.

The bispecific antibodies according to the present invention can be administered in the form of the above-described antitumor composition to mammals including human in a dose of 0,01 to 1 mg/kg/day. The dose may vary depending on the age, condition, etc. of patients. The administration of the antitumor composition can be effected by intravenous injection once a day (single administration or consecutive administration) or intermittently one to three times a week for once every two or three weeks.

The antitumor composition is expected to be useful for treating tumors such as colorectal cancer and mammary cancer.

The following examples are provided to further illustrate the present invention, but are not to be construed to limit the scope of the present invention.

### EXAMPLE 1

The anti-sialyl-Le^{a} monoclonal antibody was prepared using a hybridoma strain KM231 (AMC-462, deposited at the European Collection of Animal Cell Cultures under the accession number of ECACC 86050801), and the anti-CD3 monoclonal antibody was prepared using a hybridoma strain OKT3 (ATCC CRL8001) which has been obtained from ATCC.

### (1) Purification of monoclonal antibody

The hybridoma strains KM231 and OKT3 were respectively administered to pristane-treated female nude mice of 8 weeks of age by intraperitoneal injection at a dose of 5 to 10 x 10⁶ cells per animal. The hybridomas caused ascites tumor formation in 10 to 21 days. The ascitic fluid was collected from each ascitic fluid-carrying mouse (1 to 8 ml per animal) and centrifuged (3,000 rpm, 5 minutes) to remove solid matter. The resulting supernatant was dialyzed against a binding buffer (1.5 M glycine and 3 M NaCl, pH 9) and then applied to a protein A Sepharose column (Pharmacia). After washing the column, the IgG fraction was eluted with an elution buffer (0.1 M citrate buffer, pH 4.0) and immediately dialyzed against PBS containing 0.5 M sodium chloride. The thus dialyzed fraction was recovered to obtain purified monoclonal antibody.

When the subclass of each of the thus obtained antibodies was determined using a mouse monoclonal antibody typing kit (Zymed Laboratories), the monoclonal antibodies KM231, specific for the anti-sialyl-Le^{a} antigen, and OKT3, specific for CD3, were found to be IgG1 and IgG2a, respectively.

### (2) Preparation of bispecific antibody

The monoclonal antibody OKT3 was treated with pepsin (Sigma Chemical Co.) in 0.1 M citrate buffer (pH 4.1) at 37°C for 2 hours to prepare an F(ab')₂ fragment. The enzyme reaction was terminated by increasing the pH level to 8 with 1 M NaHCO₃. The monoclonal antibody KM231 was treated with activated papain (Worthington) in 0.1 M acetate buffer (pH 5.5) containing 3 mM EDTA at 37°C for 3 hours in the presence of cysteine to prepare an F(ab')₂ fragment. The enzyme reaction was terminated by adding iodoacetamide to a final concentration of 10 mM. Each of the two types of F(ab')₂ fragments was purified by gel chromatography.

The F(ab')₂ fragment of monoclonal antibody OKT3 was reduced by treating it with 0.5 mM DTT at pH 7.5 for 30 minutes. The reduction product was further allowed to react with DTNB (final concentration, 5 mM), and the Fab'-S-NB thus formed was purified by gel chromatography. The F(ab')₂ fragment of monoclonal antibody KM231 was reduced in the same manner with 0.5 mM DTT, and the resulting Fab'-SH was purified by gel chromatography.

Purified Fab'-S-NB and Fab'-SH were mixed together at a ratio of 1:1 and incubated at room temperature for 4 hours, and the thus reconstructed F(ab')₂ was isolated by gel chromatography to serve as the bispecific antibody in the following experiments.

### (3) Confirmation of the reactivity of bispecific antibody

The bispecific antibody prepared in the above step (2) was checked for reactivity with (a) activated CD8⁺ T cells, (b) activated CD4⁺ T cells, both of which have been isolated from human lymphocytes and activated by culturing them in the presence of the anti-CD3 antibody and IL-2, and (c) sialyl-Le^{a} antigen-positive cells LS174T, in accordance with the fluorescent antibody technique (E. Harlow and D. Lane, *Antibodies* - *A Laboratory Manual*, Cold Spring Harbor Laboratory, 1988). The reactivity was analyzed by a flow cytometry (FACScan). The results are shown in Fig. 1.

As shown in Fig. 1, the monoclonal antibody OKT3 and the bispecific antibody were capable of reacting with the activated CD8⁺ T cells or the activated CD4⁺ T cells, while the monoclonal antibody KM231 did not react with these cells. In addition, the bispecific antibody and the monoclonal antibody KM231 were capable of reacting with LS174T cells which expresses the sialyl-Le^{a} antigen at a high level, while the monoclonal antibody OKT3 did not react with these cells. Thus, it was confirmed that the bispecific antibody of the present invention reacts with both CD3-positive lymphocytes and sialyl-Le^{a} antigen-expressing cells.

### (4) Antitumor effect of bispecific antibody

The activated CD8⁺ T cells or activated CD4⁺ T cells and sialyl-Le^{a} antigen-expressing or non-expressing cells (sialyl-Le^{a} antigen-positive cells or sialyl-Le^{a} antigen-negative cells) labeled with ⁵¹Cr were cultured for four hours at a ratio of 20:1 or 10:1 in the presence or absence of the bispecific antibody (1 µg/ml) to examine the antitumor effect by measuring ⁵¹Cr gamma rays released from dead cells. The results are shown in Fig. 2 (in the case of using activated CD8⁺ T cells) and in Fig. 3 (in the case of using activated CD4⁺ T cells).

As shown in Figs. 2 and 3, each of the sialyl-Le^{a} antigen-positive cells Colo205, LS174T, NUGC2, HCL-1, KATO III and SW1116 showed a high death rate, while the sialyl-Le^{a} antigen-negative cells IMR32 showed almost no mortality. The bispecific antibody according to the present invention which comprises an anti-sialyl-Le^{a} monoclonal antibody, or binding fragment thereof and an anti-CD3 monoclonal antibody, or binding fragment thereof, is useful for the treatment of cancer.

While the invention has been described in detail and with reference to specific examples thereof, it will be apparent to one skilled in the art that various changes and modifications can be made therein without departing from the spirit and scope thereof.

## Claims

1. A bispecific antibody reactive with sialyl-Le^{a} antigen and CD3, which comprises an anti-sialyl-Le^{a} monoclonal antibody, or binding fragment thereof, and an anti-CD3 monoclonal antibody, or binding fragment thereof.

2. The bispecific antibody according to claim 1 wherein said anti-sialyl-Le^{a} monoclonal antibody is KM231, or binding fragment thereof.

3. The bispecific antibody according to claim 1 wherein said anti-CD3 monoclonal antibody is OKT3, or binding fragment thereof.

4. A pharmaceutical composition comprising at least one bispecific antibody according to one of claims 1 to 3 in an amount sufficient to kill sialyl-Le^{a} antigen expressing cells, and optionally a pharmaceutically acceptable carrier.

5. The use of at least one bispecific antibody according to one of claims 1 to 3 for preparing pharmaceutical composition for eliminating sialyl-Le^{a} antigen expressing tumor cells.
